(19) Europäisches Patentamt
European Patent Office
Office européen des brevets

(11) Veröffentlichungsnummer: **0 128 861**
**B1**

(12) # EUROPÄISCHE PATENTSCHRIFT

(45) Veröffentlichungstag der Patentschrift: **12.12.90**

(21) Anmeldenummer: **84810211.7**

(22) Anmeldetag: **03.05.84**

(51) Int. Cl.$^5$: **C 07 D 211/58,**
C 07 D 401/06,
C 07 D 401/14, C 08 K 5/34,
C 08 G 73/02, C 08 G 73/06,
C 08 G 69/26, C 08 L 77/06,
C 08 L 79/04

(54) **Polyaminamide enthaltend Polyalkylpiperidinylreste.**

(30) Priorität: **09.05.83 IT 2100583**

(43) Veröffentlichungstag der Anmeldung:
**19.12.84 Patentblatt 84/51**

(45) Bekanntmachung des Hinweises auf die Patenterteilung:
**12.12.90 Patentblatt 90/50**

(84) Benannte Vertragsstaaten:
**BE DE FR GB IT NL**

(56) Entgegenhaltungen:
**DE-A-2 636 144**
**US-A-4 104 248**
**US-A-4 232 131**

**PATENT ABSTRACTS OF JAPAN, Band 8, Nr. 30 (C-209)1467r, 8. Februar 1984; & JP - A - 58 194 931**

**Die Akte enthält technische Angaben, die nach dem Eingang der Anmeldung eingereicht wurden und die nicht in dieser Patentschrift enthalten sind.**

(73) Patentinhaber: **CIBA-GEIGY S.p.A.**
**Strada Statale 233-Km. 20,5**
**Origgio (IT)**
(84) **IT**

(73) Patentinhaber: **CIBA-GEIGY AG**
**Klybeckstrasse 141**
**CH-4002 Basel (CH)**
(84) **BE DE FR GB NL**

(72) Erfinder: **Cantatore, Giuseppe, Dr.**
**Via Generale Planelli, 68**
**I-70032 Bitonto (Bari) (IT)**

(74) Vertreter: **Stamm, Otto et al**
**Patentabteilung der CIBA-GEIGY AG Postfach**
**CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 128 861 B1

## Beschreibung

Die vorliegende Erfindung betrifft eine neue Klasse von Piperidinylreste enthaltenden polymeren Verbindungen und ihre Verwendung zum Stabilisieren von Polymeren.

Es ist bekannt, dass die physikalischen und chemischen Eigenschaften von synthetischen Polymeren stark beeinträchtigt werden, wenn diese dem Sonnenlicht oder dem Licht einer anderen UV-Lichtquelle ausgesetzt werden. Zur Verbesserung der Lichtbeständigkeit dieser Polymeren wurden verschiedene Stabilisatoren vorgeschlagen, wobei, insbesondere im Falle von Artikeln geringer Dicke, wie von Fasern, Bändern und Filmen, polymere Verbindungen, die Polyalkylpiperidylreste enthalten, als besonders geeignet bezeichnet werden, da sie sich dank dem relativ hohen Molekulargewicht durch besonders gute Extraktionsbeständigkeit auszeichnen. Produkte diese Art sind aus folgenden Patentschriften bekannt: In US-Patent 4 086 204 sind Polytriazinverbindungen, wie z.B. diejenige der Formel

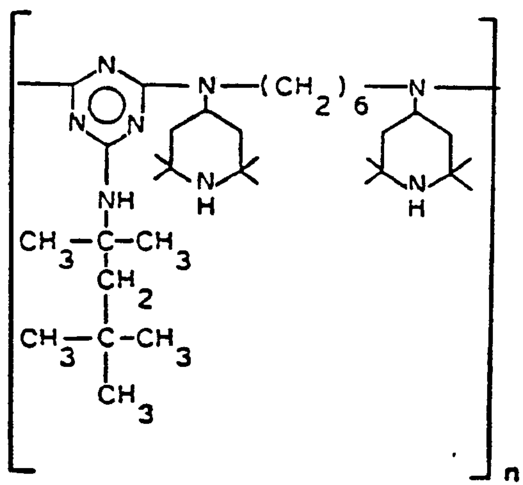

beschrieben. Polyamine, wie z.B. dasjenige der Formel

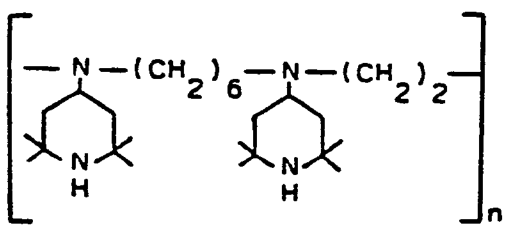

sind in US-Patent 4 104 248, und Polyamide, wie beispielsweise dasjenige der Formel

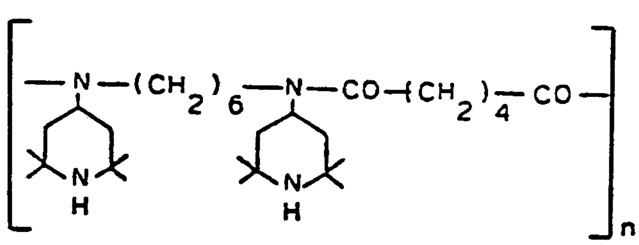

in US-Patent 4 232 131 beschrieben. Die mit diesen Produkten erhaltenen Resultate sind jedoch nicht immer ganz zufriedenstellend, so dass eine weitere Verbesserung noch wünschenswert war. Es sind nun neue Polyalkylpiperidinreste enthaltend Polyaminamide gefunden worden, die im Vergleich zu den bekannten Verbindungen eine überraschende, unvorhersehbare Verbesserung der Lichtschutzwirkung in synthetischen Polymeren zeigen.

Die vorliegende Erfindung betrifft demnach Verbindungen de allgemeinen Formel I

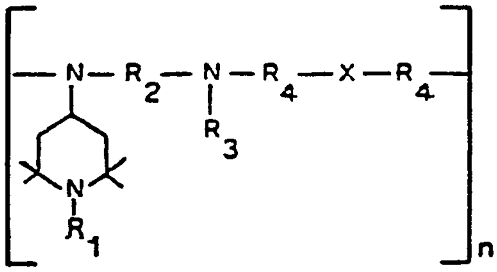

(I)

worin $R_1$ Wasserstoff, O°, $CH_2CN$, $C_1$—$C_{12}$ Alkyl, $C_3$—$C_{12}$ Alkenyl, $C_3$—$C_{12}$ Alkynyl, unsubstituiertes oder substituiertes $C_7$—$C_{12}$-Aralkyl oder $C_1$—$C_{12}$ Acyl ist, $R_2$ $C_2$—$C_{18}$ Alkylen, $C_5$—$C_{18}$ Cycloalkylen, $C_6$—$C_{18}$ Arylen oder $C_7$—$C_{18}$ Aralkylen bedeuet, $R_3$ Wasserstoff, $C_1$—$C_{18}$ Alkyl, $C_5$—$C_{18}$ Cycloalkyl, unsubstituiertes oder substituiertes $C_6$—$C_{18}$ Aryl, unsubstituiertes oder substituiertes $C_7$—$C_{18}$ Aralkyl oder ein Rest der Formel II

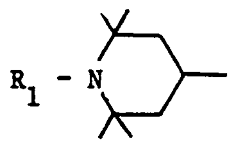

(II)

ist, worin $R_1$ die oben angegebene Bedeutung hat, beide $R_4$-Reste eine Gruppe —$C_mH_{2m}$—C(O)— bedeuten, wobei die Carbonylgruppe entweder an X oder an N gebunden ist und m eine Zahl von 1 bis 12 bedeutet, X ein zweiwertiger Rest enthaltend einen oder mehrere heterocyclische Reste der Formeln III, IV oder V

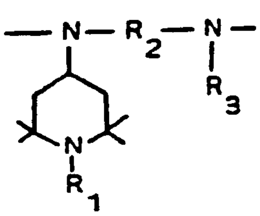

(III)

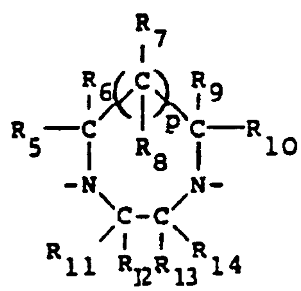

(IV)

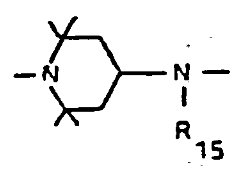

(V)

ist, worin $R_1$, $R_2$ und $R_3$ die oben angegebene Bedeutung haben, p Null oder 1 ist, $R_5$, $R_6$, $R_7$, $R_8$, $R_9$, $R_{10}$, $R_{11}$, $R_{12}$, $R_{13}$ und $R_{14}$ Wasserstoff oder Methyl bedeuten, $R_{15}$ Wasserstoff, $C_1$—$C_{18}$ Alkyl, $C_5$—$C_{18}$ Cycloalkyl, unsubstituiertes oder substituiertes $C_6$—$C_{18}$ Aryl oder $C_7$—$C_{18}$ Aralkyl oder ein Rest der Formel II ist und n eine Zahl von 2 bis 100 bedeutet.

In der vorliegenden Anmeldung bedeutet

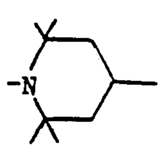

immer

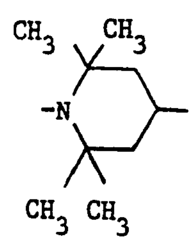

Substituiertes Aryl oder Aralkyl bedeutet durch Hydroxy und/oder durch ein oder mehrere $C_1$—$C_4$-Alkylgruppen substituiertes Aryl oder Aralkyl.

Die Bedeutungen der verschiedenen Reste speziell erläuternde Beispiele sind wie folgt:

Für $R_1$ Wasserstoff, Cyanomethyl, Methyl, Ethyl, Propyl, n-Butyl, sek. Butyl, tert.Butyl, Hexyl, Octyl, 1,1,3,3-Tetramethylbutyl, Decyl, Dodecyl, Allyl, Methallyl, But-2-enyl, Hex-2-enyl, Undec-10-enyl, Propargyl, Benzyl, 4-Methylbenzyl, 4-t-Butylbenzyl, 4-Hydroxybenzyl, Acetyl, Propionyl, Butyryl, Caproyl und Benzoyl;

für $R_2$ Ethylen, 1,2- oder 1,3-Propylen, Tetramethylen, Hexamethylen, Decamethylen, Dodecamethylen,

Cyclohexylen, Cyclohexylendimethylen, o-, m- und p-Phenylen und o-, m- und p-Xylylen;

für $R_3$ Wasserstoff, Aethyl, Ethyl, Propyl, Isopropyl, n-Butyl, sek.Butyl, tert.Butyl, Isobutyl, Hexyl, 2-Ethylhexyl, Octyl, 1,1,3,3-Tetrametylbutyl, Decyl, Dodecyl, Octadecyl, Cyclohexyl, 2- oder 4-Methylcyclohexyl, 3,3,5-Trimethylcyclohexyl, Cyclooctyl, Cyclododecyl, Phenyl, o-, m- und p-Methylphenyl, 2,6-Dimethylphenyl, 2,4,6-Trimethylphenyl, 4-t-Butylphenyl, 4-t-Octylphenyl, 4-Methoxyphenyl, 4-Ethoxyphenyl, 3,5-Di-t-butyl-4-hydroxyphenyl, Benzyl, 4-Methylbenzyl, 4-Hydroxybenzyl, 3,5-Di-t-butyl-4-hydroxybenzyl, 2,2,6,6-Tetramethyl-piperidin-4-yl, 1,2,2,6,6-Pentamethyl-piperidin-4-yl, 1-Allyl-2,2,6,6-tetramethyl-piperidin-4-yl, 1-Benzyl-2,2,6,6-tetramethyl-piperidin-4-yl und 1-Acetyl-2,2,6,6-tetramethyl-piperidin-4-yl;

für $R_{15}$ Wasserstoff, Methyl, Ethyl, Propyl, Isopropyl, Butyl, sek.Butyl, tert.Butyl, Isobutyl, Hexyl, 2-Ethylhexyl, Octyl, 1,1,3,3-Tetramethylbutyl, Decyl, Dodecyl, Hexadecyl, Octadecyl, Cyclohexyl, 2- oder 4-Methylcyclohexyl, 3,3,5-Trimethylcyclohexyl, Cyclooctyl, Cyclododecyl, Phenyl, o-, m- und p-Methylphenyl, 2,6-Dimethylphenyl, 2,4,6-Trimethylphenyl, 4-t-Butylphenyl, 4-t-Octylphenyl, 4-Methoxyphenyl, 4-Ethoxyphenyl, 3,5-Di-t-butyl-4-hydroxyphenyl, Benzyl, 4-Methylbenzyl, 4-Hydroxybenzyl, 3,5-Di-t-butyl-4-hydroxybenzyl, 2,2,6,6-Tetramethylpiperidin-4-yl, 1,2,2,6,6-Pentamethyl-piperidin-4-yl, 1-Allyl-2,2,6,6-tetramethyl-piperidin-4-yl, 1-Benzyl-2,2,6,6-tetramethyl-piperidin-4-yl und 1-Acetyl-2,2,6,6-tetramethyl-piperidin-4-yl.

Bevorzugt sind Verbindungen der Formel I, worin $R_1$ Wasserstoff, $C_1$—$C_6$ Alkyl, $C_3$—$C_6$ Alkenyl oder Alkynyl, Benzyl ode $C_1$—$C_6$ Acyl ist, $R_2$ $C_2$—$C_{12}$ Alkylen, $C_6$—$C_{10}$ Cycloalkylen oder $C_8$—$C_{10}$ Aralkylen bedeutet, $R_3$ $C_1$—$C_{12}$ Alkyl, $C_6$—$C_{10}$ Cycloalkyl oder ein Rest der Formel II ist, worin $R_1$ Wasserstoff, Methyl, Allyl, Benzyl oder Acetyl bedeutet, $R_{15}$ Wasserstoff, $C_1$—$C_{12}$ Alkyl, $C_6$—$C_{10}$ Cycloalkyl, Benzyl oder ein Rest der Formel II ist, worin $R_1$ Wasserstoff, Methyl, Allyl, Benzyl oder Acetyl bedeutet, m eine Zahl von 1 bis 5 und n eine Zahl von 2 bis 50 sind. Besonders bevorzugt sind Verbindungen der Formel I, worin $R_1$ Wasserstoff oder Methyl, $R_2$ $C_2$—$C_6$ Alkylen, $R_3$ $C_1$—$C_4$ Alkyl, Cycloalkyl, 2,2,6,6-Tetramethyl-piperidin-4-yl oder 1,2,2,6,6-Pentamethyl-piperidin-4-yl, $R_7$, $R_8$, $R_{11}$, $R_{12}$, $R_{13}$ und $R_{14}$ Wasserstoff, $R_{15}$ Wasserstoff oder $C_1$—$C_4$ Alkyl sind, m die Zahl 1 oder 2 und n eine Zahl von 2 bis 20 bedeuten.

Die Verbindungen der Formel I können durch Umsetzung eines Bis-halogenamids der Formel VI

$$Z—C_mH_{2m}\overset{\overset{\large O}{\|}}{C}—X—\overset{\overset{\large O}{\|}}{C}—C_mH_{2m}—Z \qquad (VI)$$

worin Z Chlor oder Brom ist und X und m die oben angegebene Bedeutung haben, mit einem Diamin der Formel VII

$$H—X—H \qquad (VII)$$

worin die oben angegebene Bedeutung hat, erhalten werden, mit der Bedingung, dass in mindestens einem der Zwischenprodukte VI und VII X eine Gruppe

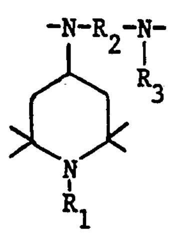

bedeutet. Die Reaktion wird in einem inerten organischen Lösungsmittel in Gegenwart einer organischen oder anorganischen Base bei Temperaturen zwischen 50 und 200°C, bevorzugt zwischen 80 und 180°C, im molaren Verhältnis VII:VI zwischen 1,5:1 und 1:1,5, bevorzugt zwischen 1,2:1 und 1:1, durchgeführt.

Geeignete Lösungsmittel zur Durchführung der obenerwähnten Umsetzung sind beispielsweise Benzol, Toluol, Xylol, Ethylbenzol, Trimethylbenzol, Tetralin, Decalin, Methanol, Ethanol, Isopropanol, n-Butanol, Butan-2-ol, Isobutanol, n-Pentanol, Isopentanol, t-Pentanol, n-Hexanol, 4-Methylpentan-2-ol, 2-Methoxyethanol, 2-Ethoxyethanol, 2-Butoxyethanol, Tetrahydrofuran, Dioxan, Dibutyläther, 1,2-Dimethoxyethan, 1,2-Diethoxyethan, Diethylenglycol-dimethyläther, Diethylenglycol-diethyläther, N,N-Dimethylformamid, N,N-Dimethylacetamid, N-Methylpyrrolidon und Dimethylsulfoxid.

Zum Abfangen des bei der Reaktion entstehenden Chlorwasserstoffs wird die Umsetzung in Gegenwart einer organischen oder anorganischen Base vorgenommen, wie z.B. Pyridin, Triethylamin, Tributylamin, Natriumhydroxid, Kaliumhydroxid, Natriumcarbonat oder Kaliumcarbonat, wobei die Base zumindest in der äquivalenten Menge in Bezug auf den sich bildenen Chlorwasserstoff eingesetz werden muss. Die Bis-halogenamide der Formel VI sind bekannt oder, sollten einige noch neu sein, können sie nach bekannten Methoden hergestellt werden, wie beispielsweise durch Umsetzung eines Halogenoacylhalogenids mit einem Diamin der Formel VII.

Zur besseren Veranschaulichung der vorliegenden Erfindung sind im folgenden einige Herstellungsbeispiele für Verbindungen der Formel I angegeben; diese Beispiele dienen ausschliesslich

der Erläuterung und bedeuten keinerlei Einschränkung der Erfindung.

Herstellung der Bis-halogenamide

N,N'-Bis-(chloracetyl)-N,N'-bis-(2,2,6,6-tetramethyl-piperidin-4-yl)-1,6-diaminohexan

Eine Lösung von 118,6 g (1,05 Mol) Chloracetylchlorid in 100 ml Methylenchlorid wird langsam einer auf −20°C abgekühlten Lösung von 197 g (0,5 Mol) N,N'-Bis-(2,2,6,6-tetramethyl-piperidin-4-yl)-1,6-diaminohexan in 1000 ml Methylenchlorid zugegeben, wobei die Temperatur −10°C nicht übersteigen soll. Die Reaktionsmischung wird eine Stunde gerührt und die Temperatur in dieser Zeit allmählich auf 0°C steigen gelassen. Danach wird innerhalb einer Stunde eine Lösung von 44 g (1.1 Mol) Natriumhydroxid in 180 ml Wasser zugegeben und eine weitere Stunde bei Zimmertemperatur gerührt. Dann wird die wässrige Phase abgetrennt, die organische Phase zur Trockne eingedampft und der Rückstand aus Aceton umkristallisiert. Das erhaltene Produkt hat einen Smp. von 131°C.

Folgende Bis-halogenamide können durch analoges Verfahren erhalten werden:

| Bis-halogenoamid | umkristallisiert aus | Smp. (°C) |
|---|---|---|
| 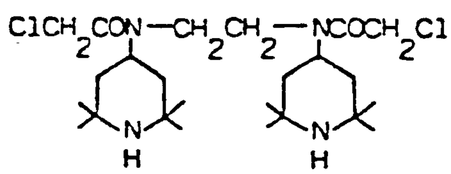 | 1,2-Dichlorethan | 223-5 |
| 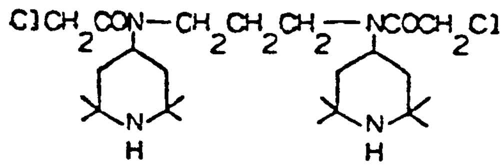 | Aceton | 123 |
| 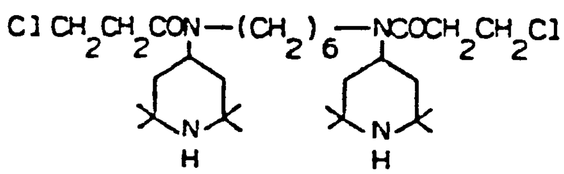 | Isopropanol | 146 |
| 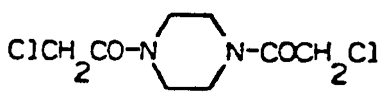 | Ethanol | 137 |
| 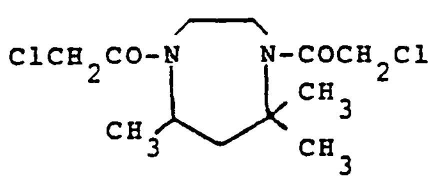 | Aceton | 83-5 |
| 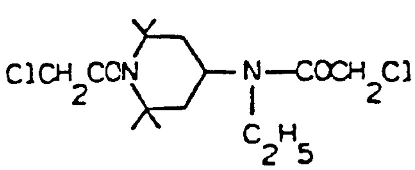 | Aceton/ Hexan (1:2) | 79-80 |

## Beispiel 1

54,7 g (0.1 Mol) N,N'-Bis-(chloracetyl)-N,N'-bis(2,2,6,6-tetramethyl-piperidin-4-yl)-1,6-diaminohexan, 33,8 g (0,1 Mol) N,N'-bis-(2,2,6,6-tetramethyl-piperidin-4-yl)-1,2-diaminoethan, 8,4 g Natriumhydroxid und 100 ml Xylol werden vermischt und 12 Stunden unter Rückfluss gekocht und das sich bildende Wasser azeotrop abdestilliert. Nach dem Abkühlen auf 80°C werden die anorganischen Produkte abfiltriert und das Filtrat zur Trockne eingedampft. Zu diesem Zweck wird vorerst bei Normaldruck auf 160°C geheizt und dann bei Beibehaltung der gleichen Temperatur Vakuum angesetzt (1,3 mbar). Man erhält ein gelbiches harziges Produkt mit Smp. 115—123°C und mittlerem Molekulargewicht $\overline{M}n$ 440.

## Beispiele 2—11

Wiederholt man das unter Beispiel 1 beschriebene Verfahren mit entsprechendem Bis-chloramid (VI) und Diamin (VII), so erhält man folgende Polyaminoamide der Formel

$$-\!\left[CH_2\overset{\overset{O}{\|}}{C}-A-\overset{\overset{O}{\|}}{C}-CH_2-B\right]_n-$$

| Beispiel | A | B | Smp. | $\bar{M}n$ |
| --- | --- | --- | --- | --- |
| 2 | 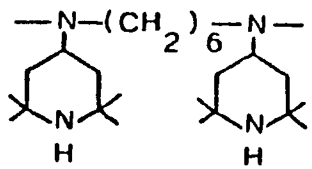 | 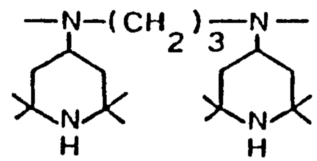 | 103-110 | 2500 |
| 3 | 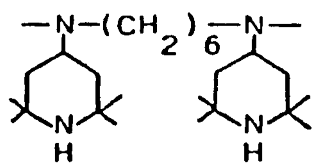 | 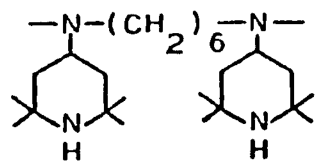 | 95-106 | 4350 |
| 4 | 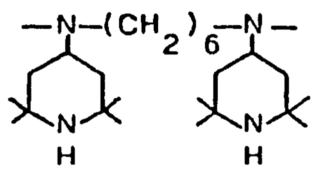 | 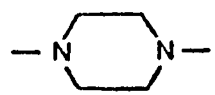 | 180-194 | 5600 |

| Beispiel | A | B | Smp. | $\bar{M}n$ |
|---|---|---|---|---|
| 5 | 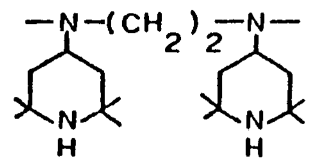 | 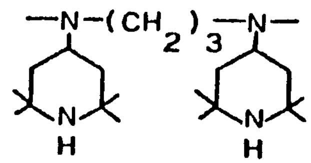 | 130-138 | 2570 |
| 6 | 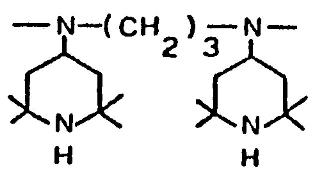 | 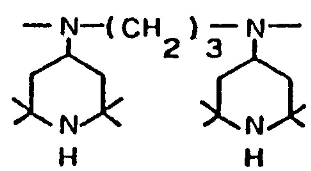 | 125-135 | 3930 |
| 7 | 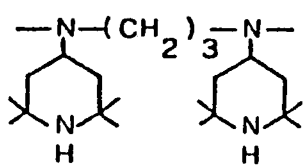 | 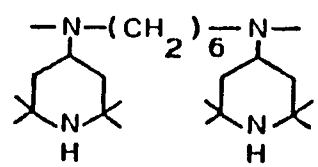 | 102-107 | 2470 |

| Beispiel | A | B | Smp. | $\bar{M}n$ |
|---|---|---|---|---|
| 8 | 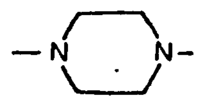 | 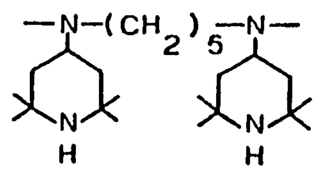 | 137-144 | 4180 |
| 9 | 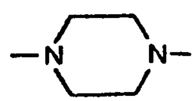 | 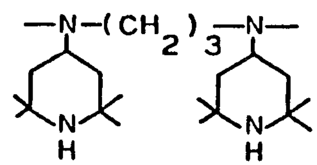 | 95-104 | 2470 |

| Beispiel | A | B | Smp. | $\bar{M}n$ |
|---|---|---|---|---|
| 10 | 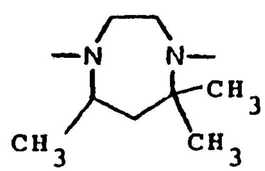 | 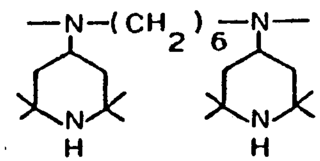 | 80-87 | 2300 |
| 11 | 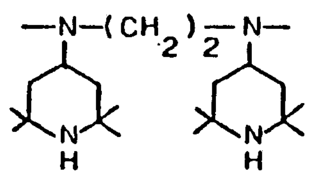 | 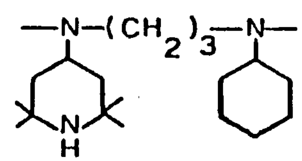 | 140-150 | 3700 |

Beispiel 12

49, 1 g (0,1 Mol) N,N'-Bis-(chloracetyl)-N,N'-bis-(2,2,6,6-tetramethyl-piperidin-4-yl)-1,2-diaminoethan, 39,4 g (0,1 Mol) N,N'-Bis-(2,2,6,6-tetramethyl-piperidin-4-yl)-1,6-diaminohexan, 8,4 g Natriumhydroxid und 200 ml 2-Ethoxyethanol werden vermischt und 16 Stunden unter Rückfluss gekocht. Danach werden die anorganischen Produkte abfiltriert und das Lösungsmittel abgedampft. Man erhält eine gelbliche harzige feste Substanz mit Smp. 120—129°C un $\overline{M}n$ 2200.

Beispiel 13

33,7 g (0,1 Mol) 1-Chloracetyl-4-(N-ethylchloracetamido)-2,2,6,6-tetramethyl-piperidin, 39,4 g (0,1 Mol) N,N'-Bis-(2,2,6,6-tetramethyl-piperidin-4-yl)-1,6-diaminohexan, 100 ml N,N-Dimethylformamid und 27,6 g (0,2 Mol) Kaliumcarbonat werden vermischt und 16 Stunden auf 100°C geheizt. Danach werden die unlöslichen Produkte durch Filtration abegrennt und das Lösungsmittel abgedampft. Man erhält eine harzige feste Substanz mit Smp. 80—92°C und $\overline{M}n$ 1800.

Beispiel 14

54,7 g (0,1 Mol) N,N'-Bis-(3-chlorpripionyl)-N,N'-bis-(2,2,6,6-tetramethyl-piperidin-4-yl)-1,6-diaminohexan, 8,6 g (0,1 Mol) Piperazin und 150 ml Methylisobutylcarbinol werden vermischt und 4 Stunden unter Rückfluss gekocht. Nachdem 8 g Natriumhydroxid zugegeben worden sind, wird für weitere 6 Stunden unter Rückfluss gekocht. Dann werden die anorganischen Produkte durch Filtration abgetrennt und das Lösungsmittel abgedampft. Man erhält eine gelbliche feste Substanz mit Smp. 87—95°C und $\overline{M}n$ 2000.

Beispiel 15

50 g der Verbindung von Beispiel 13, 13,8 g Paraformaldehyd, 24 g 88%iger Ameisensäure und 100 ml Wasser werden vermischt und 10 Stunden unter Rückfluss gekocht. Nach dem Abkühlen wird eine Lösung von 18 g Natriumhydroxid in 200 ml Wasser zugegeben und die Reaktionsmischung eine Stunde bei Zimmertemperatur gerührt. Das ausgefallene Produkt wird abfiltriert, mit Wasser gewaschen und getrocknet. Man ethält ein Produkt mit Smp. 92—100°C und $\overline{M}n$ 4500.

Wie anfangs erwähnt, sind die Verbindungen der Formel I sehr wirksam bei der Verbesserung der Licht-, Wärme- und Oxydationsbeständigkeit von synthetischen Polymeren wie beispielsweise Polyäthylen hoher oder niedriger Dichte, Polypropylen, Aethylen/Propylencopolymeren, Aethylen/Vinylacetatcopolymeren, Polybutadien, Polyisopren, Polystyrol, Butadien/Styrolcopolymeren, Acralnitril/Butadien/Styrolcopolymeren, Polymeren und Copolymeren von Vinylchlorid und Vinylidenchlorid, Polyoxymethylen, Polyurethanen, gesättigten und ungesättigten Polyestern, Polyamiden, Polycarbonaten, Polyacrylaten, Alkydharzen und Epoxidharzen.

Die Verbindungen der Formel I können im Gemisch mit synthetischen Polymeren in verschiedenen Anteilen eingesetzt werden, die von der Art des Polymeren, dem Endzweck und der Gegenwart weiterer Zusatzstoffe abhängen. Im allgemeinen setzt man zweckmässig 0,01 bis 5 Gew.-% der Verbindungen der Formel I, bezogen auf das Gewicht der Polymeren, vorzugsweise 0,1 bis 1%, ein.

Die Verbindungen der Formel I können nach verschiedenen Verfahren in die polymeren Materialien eingearbeitet werden, wie durch trockenes Einmischen in Pulverform oder nasses Einmischen in Form einer Lösung oder Suspension oder auch in Form eines konzentrierten Vorgemisches; das synthetische Polymer lässt sich dabei in Form von Pulver, Granulat, Lösung, Suspension oder Latex einsetzen. Die mit den Produkten der Formel I stabilisierten Polymeren lassen sich zur Herstellung von Formkörpern, Folien, Bändern, Endlosfäden, Lacken usw. verwenden.

Gegebenenfalls kann man den Mischungen von Verbindungen der Formel I mit den synthetischen Polymeren weitere Zusatzstoffe beigeben, wie beispielsweise Antioxydantien, UV-absorbierende Stoffe, Nickelstabilisatoren, Pigmente, Füllstoffe, Weichmacher, Antistatika, Flammschutzmittel, Schmierstoffe, Korrosionshemmstoffe sowie Metalldesaktivatoren. Beispiel für in Mischung mit Verbindungen der Formel I verwendbare Zusatzstoffe sind insbesondere:

1. Antioxidantien

*1.1. Alkylierte Monophenole*

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

*1.2. Alkylierte Hydrochinone*
2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

*1.3. Hydroxylierte Thiodiphenylether*
2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

*1.4. Alkyliden-Bisphenole*
2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

*1.5 Benzylverbindungen*
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

*1.6. Acylaminophenole*
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

*1.7. Ester der β-(3,5-Di-tert.butyl-4-hydroxyphenyl)-propionsäure* mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

*1.8. Ester der β-(5-tert.butyl-4-hydroxy-3-methylphenyl)-propionsäure* mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

*1.9. Amide der β-(3,5-Di-tert.butyl-4-hydroxyphenyl)-propionsäure,* wie z.B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

2. UV-Absorber und Lichtschutzmittel

*2.1. 2-(2'-Hydroxyphenyl)-benztriazole,* wie. z.B. das 5'-Methyl-, 3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert.butyl-, 5'-methyl-, 3'-sec.Butyl-5'-tert.butyl-, 4'-Octoxy-, 3',5'-Di-tert.amyl-, 3',5'-Bis-(α,α-dimethylbenzyl)-Derivat.

2.2. *2-Hydroxybenzophenone,* wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

*2.3. Ester von gegebenenfalls substituierten Benzoesäuren,* wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxsybenzoesäure-2,4-di-tert.butylphenylester, 3,5-Di-tert.butyl-4-hydroxybenzoesäurehexadecylester.

*2.4. Acrylate,* wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

*2.5. Nickelverbindungen,* wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

*2.6. Sterisch gehinderte Amine,* wie. z.B. Bis-(2,2,6,6-tetramethylpiperidyl)-sebacat Bis-(1,2,2,6,6-pentamethylpiperidyl)-sebacat n-Butyl-3,5-di-tert.butyl-4-hydroxybenzyl-malonsäure-bis(1,2,2,6,6-penta-methylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)nitrilo-triacetat, Tetrakis-(2,2,6,6-tetramethyl-4-piperidyl)-1,2,3,4-butantetracarbonsäure, 1,1'-(Ethandiyl)-bis(3,3,5,5-tetramethyl-piperazinon).

*2.7. Oxalsäurediamide,* wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von ortho-und para-Methoxy- sowie von o- und p-Ethoxy-disubstituierte Oxaniliden.

*3. Metalldesaktivatoren,* wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropinyl)-hydrazin, 3-Salicyloylamino-1,2,4-triazol, Bis-benzyliden-oxalsäuredihydrazid.

*4. Phosphite und Phosphonite,* wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit.

*5. Peroxidzerstörende Verbindungen,* wie z.B. Ester der β-Thio-dipropionsäure, beispielsweise der Lauryl-, Stearyl-, Myristyl- oder Tridecylester, Mercaptobenzimidazol, das Zinksalz des 2-Mercaptoabenzimidazols, Zink-dibutyl-dithiocarbamat, Dioctadecyldisulfid, Pentaerythrit-tetrakis-[β-dodecylmercapto)-propionat.

*6. Polyamidstabilisatoren,* wie z.B. Kupfersalze in Kombination mit Jodiden und/oder Phosphorverbindungen und Salze des zweiwertigen Mangans.

*7. Basische Co-Stabilisatoren,* wie z.B. Melamin, Polyvinylpyrrolidon, Dicyandiamid, Triallylcyanurat, Harnstoff-Derivat, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

*8. Nukleierungsmittel,* wie z.B. 4-tert.Butylbenzoesäure, Adipinsäure, Diphenylessigsäure.

*9. Füllstoffe und Verstärkungsmittel,* wie z.B. Calciumcarbonat, Silikate, Glasfasern, Asbest, Talk,

Kaolin, Glimmer, Bariumsulfat, Metalloxide und -hydroxide, Russ, Graphit.

*10. Sonstige Zusätze,* wie z.B. Weichmacher, Gleitmittel, Emulgatoren, Pigmente, Optische Aufheller, Flammschutzmittel, Antistatika, Treibmittel.

Die Wirksamkeit der Erfindungsgemässen Produkte als Stabilisatoren wird in den nachfolgenden Beispielen veranschaulicht, in welchen die Resultate der Stabilisierung von Polypropylenbändern und -fasern mit einigen typischen erfindungsgemässen Polyaminamiden wiedergegeben werden.

Beispiel 16

Je 2 g der in Tabelle 1 angeführten Verbindungen und 1 g Pentaerythrit-tetrakis-3-(3,5-di-t-butyl-4-hydroxyphenyl)-propionat (Antioxydans) werden mit 1000 g Polypropylen mti einem Schmelzindex von 2,4 (® Propathen HF 18, Produkt der Imperial Chemical Industries) und 1 g Calciumstearat innig vermischt.

Das erhaltene Gemisch wird dann bei einer Temperatur von 180—220°C extrudiert und zu Granulat verarbeitet, aus dem man Bänder einer Dicke von 50 µm und einer Breite von 2,5 mm herstellt. Die Arbeitsbedingungen sind:

Extrudertemperatur: 220—240°C
Kopftemperatur: 240°C
Streckverhältnis: 1:6

Die erhaltenen Bänder werden auf weisser Pappe angeordnet in einem Weather-Ometer 65 WR(ASTM G 27—70) bei einer Temperatur der schwarzen Tafel von 63°C ausgesetzt. Von Zeit zu Zeit werden Proben entnommen, an denen man die verbleibende Zugfestigkeit mittels eines Tensometers mit konstanter Geschwindigkeit misst; danach bestimmt man die zum Halbieren der ursprünglichen Zugfestigkeit erforderliche Ausstzungszeit ($T_{50}$).

Die Resultate sind in Tabelle 1 wiedergegeben.

TABELLE 1

| Stabilisator | $T_{50}$ (Stunden) |
|---|---|
| keiner | 230 |
| Verbindung von Bsp. 1 | 2350 |
| Verbindung von Bsp. 2 | 2220 |
| Verbindung von Bsp. 3 | 1700 |
| Verbindung von Bsp. 5 | 2130 |
| Verbindung von Bsp. 6 | 1870 |
| Verbindung von Bsp. 7 | 2350 |
| Verbindung von Bsp. 10 | 2120 |
| Verbindung von Bsp. 12 | 2000 |

Beispiel 17

Je, 2,5 g der in Tabelle 2 angeführten Verbindungen und 1 g n-Octadecyl-3-(3,5-di-t-butyl-4-hydroxyphenyl)-propionat (Antioxidans) werden mit 1000 g Polypropylen mit einem Schmelzindex von 13 (® Propathen HF 85, Produkt der Imperial Chemical Industries), 1 g Calciumstearat und 1 g Titandioxid innig vermischt.

Das erhaltene Gemisch wird dann bei einer Temperatur von 180—220°C extrudiert und zu Granulat verarbeitet. Die so erhaltenen Granulate werden unter folgenden Bedingungen zu Fasern (Titer: 20 den/Faser) verarbeitet:

Extrudertemperatur: 220—240°C
Kopftemperatur: 240°C
Streckverhältnis: 1:3,5.

Die erhaltenen Fasern werden auf weisser Pappe angeordnet in einem Weather-Ometer 65 WR bei einer Temperatur der schwarzen Tafel von 63°C ausgesetzt. Der $T_{50}$-Wert wird wie unter Beispiel 16 beschrieben bestimmt.

Die Resultate sind in Tabelle 2 wiedergegeben.

TABELLE 2

| Stabilisator | $T_{50}$ (Stunden) |
|---|---|
| keiner | 190 |
| Verbindung von Bsp. 1 | 1560 |
| Verbindung von Bsp. 2 | 1370 |
| Verbindung von Bsp. 3 | 1310 |
| Verbindung von Bsp. 5 | 1400 |
| Verbindung von Bsp. 6 | 1200 |
| Verbindung von Bsp. 7 | 1800 |
| Verbindung von Bsp. 8 | 1320 |
| Verbindung von Bsp. 12 | 1560 |

## Patentansprüche

1. Verbindung der Formel I

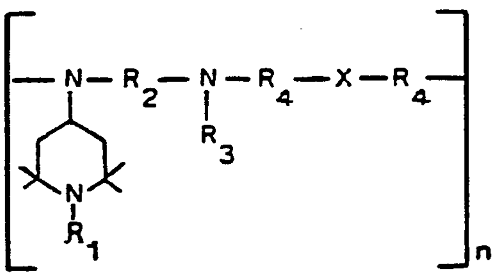

(I)

worin $R_1$ Wasserstoff, O°, $CH_2CN$, $C_1$—$C_{12}$ Alkyl, $C_3$—$C_{12}$ Alkenyl, $C_3$—$C_{12}$ Alkynyl, unsubstituiertes oder substituiertes $C_7$—$C_{12}$-Aralkyl oder $C_1$—$C_{12}$ Acyl ist, $R_2$ $C_2$—$C_{18}$ Alkylen, $C_5$—$C_{18}$ Cycloalkylen, $C_6$—$C_{18}$ Arylen oder $C_7$—$C_{18}$ Aralkylen bedeuet, $R_3$ Wasserstoff, $C_1$—$C_{18}$ Alkyl, $C_5$—$C_{18}$ Cycloalkyl, unsubstituiertes oder substituiertes $C_6$—$C_{18}$ Aryl, unsubstituiertes oder substituiertes $C_7$—$C_{18}$ Aralkyl oder ein Rest der Formel II

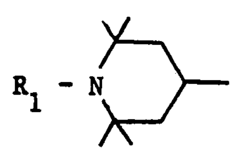

(II)

ist, worin $R_1$ die oben angegebene Bedeutung hat, beide $R_4$-Reste eine Gruppe $—C_mH_{2m}—C(O)—$ bedeuten, wobei die Carbonylgruppe entweder an X oder an N gebunden ist und m eine Zahl von 1 bis 12 bedeutet, X ein zweiwertiger Rest enthaltend einen oder mehrere heterocyclische Reste der Formeln III, IV oder V

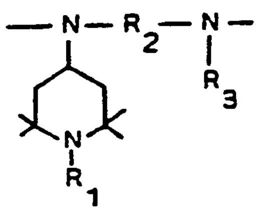

(III)

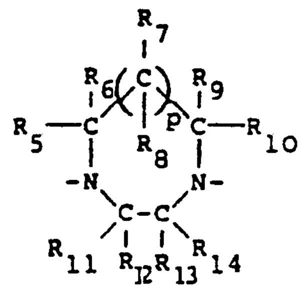

(IV)

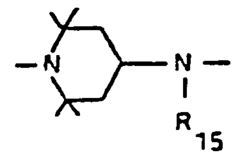

(V)

ist, worin $R_1$, $R_2$ und $R_3$ die oben angegebene Bedeutung haben, p Null oder 1 ist, $R_5$, $R_6$, $R_7$, $R_8$, $R_9$, $R_{10}$, $R_{11}$, $R_{12}$, $R_{13}$ und $R_{14}$ Wasserstoff oder Methyl bedeuten, $R_{15}$ Wasserstoff, $C_1—C_{18}$ Alkyl, $C_5—C_{18}$ Cycloalkyl, unsubstituiertes oder substituiertes $C_6—C_{18}$ Aryl oder $C_7—C_{18}$ Aralkyl oder ein Rest der Formel II ist und n eine Zahl von 2 bis 100 bedeutet.

2. Verbindung gemäss Anspruch 1, der Formel I, worin $R_1$ Wasserstoff, $C_1—C_6$ Alkyl, $C_3—C_6$ Alkenyl oder Alkynyl, Benzyl oder $C_1—C_6$ Acyl ist, $R_2$ $C_2—C_{12}$ Alkylen, $C_6—C_{10}$ Cycloalkylen oder $C_8—C_{10}$ Aralkylen bedeutet, $R_3$ $C_1—C_{12}$ Alkyl, $C_6—C_{10}$ Cycloalkyl oder ein Rest der Formel II ist, worin $R_1$ Wasserstoff, Methyl, Allyl, Benzyl oder Acetyl bedeutet, $R_{15}$ Wasserstoff, $C_1—C_{12}$ Alkyl, $C_6—C_{10}$ Cycloalkyl, Benzyl oder ein Rest der Formel II ist, worin $R_1$ Wasserstoff, Methyl, Allyl, Benzyl oder Acetyl bedeutet, m eine Zahl von 1 bis 5 und n eine Zahl von 2 bis 50 sind.

3. Verbindung gemäss Anspruch 1, der Formel I, worin $R_1$ Wasserstoff oder Methyl, $R_2$ $C_2—C_6$ Alkylen, $R_3$ $C_1—C_4$ Alkyl, Cyclohexyl, 2,2,6,6-Tetramethyl-piperidin-4-yl oder 1,2,2,6,6-Pentamethyl-piperidin-4-yl, $R_7$, $R_8$, $R_{11}$, $R_{12}$, $R_{13}$ und $R_{14}$ Wasserstoff, $R_{15}$ Wasserstoff oder $C_1—C_4$ Alkyl sind, m die Zahl 1 oder 2 und n eine Zahl von 2 bis 20 bedeuten.

4. Polymerzusammensetzungen, dadurch gekennzeichnet, dass sie einen synthetischen Polymeren und eine Stabilisatorverbindung der Formel I gemäss Anspruch 1 in einer Menge zwischen 0,01 und 5 Gew.-%, bezogen auf das Gewicht des synthetischen Polymeren, enthalten.

5. Zusammensetzungen nach Anspruch 4, dadurch gekennzeichnet, dass sie zusätzlich zu dem neuen Stabilisator weitere übliche Zusatzstoffe für synthetische Polymere enthalten.

6. Zusammensetzungen nach Anspruch 4, dadurch gekennzeichnet, dass als synthetisches Polymer Polyäthylen oder Polypropylen vorliegt.

**Revendications**

1. Composés de formule I

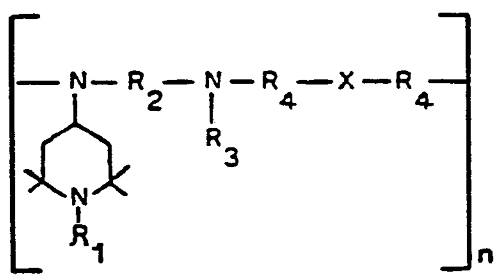

(I)

dans laquelle

$R_1$ désigne l'hydrogène, O°, le groupe $CH_2CN$, un alkyle en $C_1—C_{12}$, un alcényle ou un alcynyle en $C_3—C_{12}$, un aralkyle en $C_7—C_{12}$ avec ou sans substituants ou un acyle en $C_1—C_{12}$,

$R_2$ un alkylène en $C_2—C_{18}$, un cycloalkylène en $C_5—C_{18}$, un arylène en $C_6—C_{18}$ ou un aralkylène en $C_7—C_{18}$,

$R_3$ l'hydrogène, un alkyle en $C_1$—$C_{18}$, un cycloalkyle en $C_5$—$C_{18}$, un aryle en $C_6$—$C_{18}$ avec ou sans substituants, un aralkyle en $C_7$—$C_{18}$ avec ou sans substituants ou encore un radical de formule II:

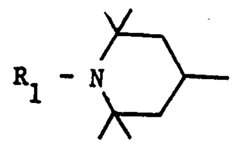

(II)

$R_1$ ayant la même signification que ci-dessus, les deux radicaux $R_4$ désignent chacun un groupe —$C_mH_{2m}$—C(O)— dont le groupe carbonyle est lié à X ou à N et m étant un nombre de 1 à 12, X est un radical divalent comportant un ou plusieurs radicaux hétérocyliques de formule III, IV ou V ci-dessous:

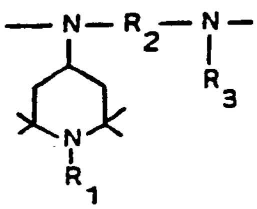

(III)

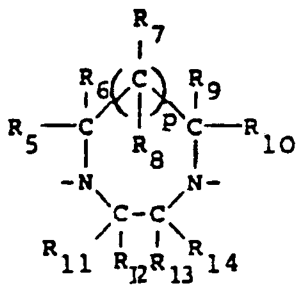

(IV)

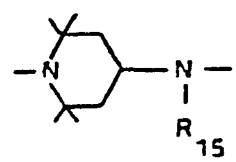

(V)

$R_1$, $R_2$ et $R_3$ ayant les significations précédentes, p étant le nombre 0 ou 1, $R_5$, $R_6$, $R_7$, $R_8$, $R_9$, $R_{10}$, $R_{11}$, $R_{12}$, $R_{13}$ et $R_{14}$ étant l'hydrogène ou un méthyle et $R_{15}$ l'hydrogène, un alkyle en $C_1$—$C_{18}$, un cycloalkyle en $C_5$—$C_{18}$, un aryle en $C_6$—$C_{18}$ avec ou sans substituants ou un aralkyle en $C_7$—$C_{18}$, ou encore un radical de formule II ci-dessus, et n est un nombre de 2 à 100.

2. Composés de formule I selon la revendication 1 dans lesquels $R_1$ désigne l'hydrogène, un alkyle en $C_1$—$C_6$, un alcényle ou un alcynyle ou $C_3$—$C_6$, un benzyle ou un acyle en $C_1$—$C_6$, $R_2$ un alkylène en $C_2$—$C_{12}$, un cycloalkylène en $C_6$—$C_{10}$ ou un aralkylène en $C_8$—$C_{10}$, $R_3$ un alkyle en $C_1$—$C_{12}$, un cycloalkyle en $C_6$—$C_{10}$ ou un radical de formule II dans lequel $R_1$ est l'hydrogène ou un groupe méthyle, allyle, benzyle ou acétyle, $R_{15}$ désigne l'hydrogène, un alkyle en $C_1$—$C_{12}$, un cycloalkyle en $C_6$—$C_{10}$, un benzyle ou un radical de formule II dans lequel $R_1$ est l'hydrogène ou le groupe méthyle, allyle, benzyle ou acétyle, m est un nombre de 1 à 5 et n un nombre de 2 à 50.

3. Composés de formule I selon la revendication 1 dans lesquels $R_1$ et l'hydrogène ou le groupe méthyle, $R_2$ un alkylène en $C_2$—$C_6$, $R_3$ un alkyle en $C_1$—$C_4$, un cyclohexyle ou un groupe 2,2,6,6-tétraméthylepipéridine-4-yle ou 1,2,2,6,6-pentaméthyl-pipéridine-4-yle, $R_7$, $R_8$, $R_{11}$, $R_{12}$, $R_{13}$ et $R_{14}$ sont l'hydrogène, $R_{15}$ et l'hydrogène ou un alkyle en $C_1$—$C_4$ m le nombre 1 ou 2 et n un nombre de 2 à 20.

4. Compositions de matières polymères caractérisées en ce qu'elles comprennent un polymère synthétique avec un composé stabilisant de formule I selon la revendication 1 dans une proportion de 0,01 à 5% du poids du polymère.

5. Compositions selon la revendication 4, caractérisées en ce que, en plus des nouveaux stabilisants de cette invention, elles contiennent également d'autres additifs qui sont couramment employés avec des matières polymères synthétiques.

6. Compositions selon la revendication 4, caractérisées en ce que le polymère synthétique est du polyéthylène ou du polypropylène.

**Claims**

1. A compound of the formula I

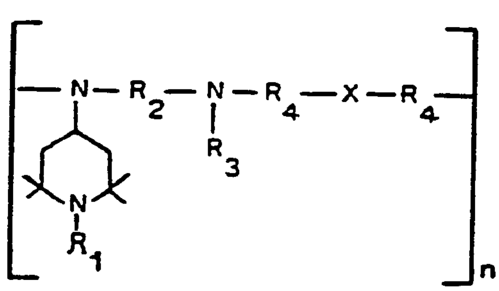

(I)

in which $R_1$ is hydrogen, O°, $CH_2CN$, $C_1$—$C_{12}$alkyl, $C_3$—$C_{12}$alkenyl, $C_3$—$C_{12}$alkynyl, substituted or unsubstituted $C_7$—$C_{12}$aralkyl or $C_1$—$C_{12}$acyl, $R_2$ is $C_2$—$C_{18}$alkylene, $C_5$—$C_{18}$cycloalkylene, $C_6$—$C_{18}$arylene or $C_7$—$C_{18}$aralkylene, $R_3$ is hydrogen, $C_1$—$C_{18}$alkyl, $C_5$—$C_{18}$cycloalkyl, substituted or unsubstituted $C_6$—$C_{18}$aryl, substituted or unsubstituted $C_7$—$C_{18}$aralkyl or a radical of the formula II

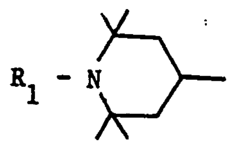

(II)

in which $R_1$ is as defined above, both radicals $R_4$ are a $—C_mH_{2m}$-C(O)-group, the carbonyl group being attached either to X or to N and in which m is a number from 1 to 12, X is a divalent radical containing one or more heterocyclic radicals of the formulae III, IV or V

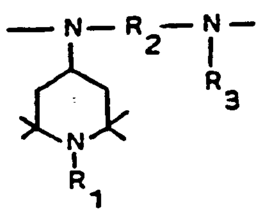

(III)

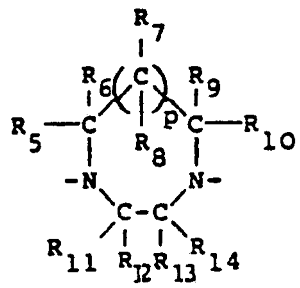

(IV)

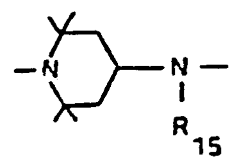

(V)

in which $R_1$, $R_2$ and $R_3$ are as defined above, p is zero or 1, $R_5$, $R_6$, $R_7$, $R_8$, $R_9$, $R_{10}$, $R_{11}$, $R_{12}$, $R_{13}$, and $R_{14}$ are hydrogen or methyl, $R_{15}$ is hydrogen, $C_1$—$C_{18}$alkyl, $C_5$—$C_{18}$cycloalkyl, substituted or unsubstituted $C_6$—$C_{18}$aryl or $C_7$—$C_{18}$aralkyl or a radical of the formula II, and n is a number from 2 to 100.

2. A compound according to claim 1, of the formula I, in which $R_1$ is hydrogen, $C_1$—$C_6$alkyl, $C_3$—$C_6$alkenyl, $C_3$—$C_6$alkynyl, benzyl or $C_1$—$C_6$acyl, $R_2$ is $C_2$—$C_{12}$alkylene, $C_6$—$C_{10}$cycloalkylene or $C_8$—$C_{10}$aralkylene, $R_3$ is $C_1$—$C_{12}$alkyl, $C_6$—$C_{10}$cycloalkyl or a radical of the formula II in which $R_1$ is hydrogen, methyl, allyl, benzyl or acetyl, $R_{15}$ is hydrogen, $C_1$—$C_{12}$alkyl, $C_6$—$C_{10}$cycloalkyl, benzyl or a radical of the formula II in which $R_1$ is hydrogen, methyl, allyl, benzyl or acetyl, m is a number from 1 to 5 and n is a number from 2 to 50.

3. A compound according to claim 1, of the formula I, in which $R_1$ is hydrogen or methyl, $R_2$ is $C_2$—$C_6$alkylene, $R_3$ is $C_1$—$C_4$alkyl, cyclohexyl, 2,2,6,6-tetramethyl-piperidin-4-yl or 1,2,2,6,6-pentamethyl-piperidin-4-yl, $R_7$, $R_8$, $R_{11}$, $R_{12}$, $R_{13}$ and $R_{14}$ are hydrogen, $R_{15}$ is hydrogen or $C_1$—$C_4$alkyl, m is the number 1 or 2 and n is a number from 2 to 20.

4. A polymer composition which contains a synthetic polymer and a stabilizer compound of the formula I, according to claim 1, in an amount between 0.01 and 5% by weight, relative to the weight of the synthetic polymer.

5. A compound according to claim 4, which, in addition to the new stabilizer, contains other conventional additives for synthetic polymers.

6. A compound according to claim 4, wherein the synthetic polymer is polyethylene or polypropylene.